# FASCICULE DE BREVET EUROPEEN

(11) **EP 3 302 650 B1**
(45) Date de publication et mention de la délivrance du brevet: **16.09.2020**
(21) Numéro de dépôt: 16726566.9
(22) Date de dépôt: 01.06.2016
(51) Int. Cl.: A61M 5/24

(54) **BOITIER DE MONTAGE D'UN RECIPIENT SUR UN STYLO INJECTEUR**
AMPULLENGEHÄUSE FÜR EINEN INJEKTIONSPEN
CONTAINER HOUSING OF A PEN INJECTOR

(30) Priorité: 02.06.2015 FR 1554993
(43) Date de publication de la demande: 11.04.2018
(73) Titulaire: Biocorp Production, 63500 Issoire (FR)
(72) Inventeur: ANEAS, Antoine, 63200 Menetrol (FR)
(74) Mandataire: Lavoix
(86) Numéro de dépôt international: PCT/EP2016/062395
(87) Numéro de publication internationale: WO 2016/193314

(56) Documents cités:
- WO-A1-2011/092326
- WO-A2-2012/017063
- US-A1- 2014 358 093

## Description

L'invention a trait à un boîtier de montage d'un récipient de forme allongée sur un stylo injecteur. L'invention a également trait à un ensemble formant réservoir de produit injectable pour un stylo injecteur, ainsi qu'à un stylo injecteur équipé d'un tel ensemble.

L'invention relève du domaine des stylos injecteurs utilisés pour injecter un principe actif par voie parentérale, c'est-à-dire par voie intraveineuse, sous-cutanée ou intramusculaire, dans le corps d'un patient. Un tel stylo peut être utilisé pour traiter un patient atteint d'une maladie spécifique, telle que le diabète ou le nanisme. En effet, le traitement d'un tel patient nécessite au moins une injection par jour, d'insuline dans le cas du diabète ou d'hormone de croissance dans le cas du nanisme. Au vu du nombre d'injections nécessaires, les patients effectuent souvent leurs injections eux-mêmes, sans faire appel à un praticien.

Pour ce faire, les patients utilisent de plus en plus souvent un stylo injecteur qui est plus facile d'utilisation qu'une seringue classique car il peut être stocké de façon discrète et sûre. Un tel stylo injecteur est mis en oeuvre avec des récipients, le plus souvent dénommées « carpules », de contenance relativement faible et permettant toutefois de réaliser un certain nombre d'injections. Une carpule est un récipient cylindrique à section circulaire, dont la longueur est de l'ordre de 60 mm et dont le diamètre extérieur est compris entre 8,5 et 12 mm, de préférence de l'ordre de 11 mm. Un tel stylo injecteur est facilement transportable par le patient, comme un stylo classique.

Pour utiliser un tel stylo injecteur, celui-ci doit être équipé d'un récipient contenant le principe actif à injecter, à savoir une capsule ou carpule comme évoqué ci-dessus. Avant chaque utilisation, une aiguille doit être montée à l'extrémité distale du récipient, cette aiguille étant jetée après utilisation, alors que le récipient demeure sur le stylo injecteur. Il convient donc de maintenir en position le récipient sur une embase du stylo injecteur.

Pour ce faire, il est connu de US-A-2014/0358093 de prévoir, à l'avant de l'embase d'un stylo injecteur, un support de cartouche adapté pour recevoir une cartouche pleine de principe actif à injecter. L'immobilisation de chaque cartouche dans ce support a lieu au moyen d'un élément de couplage qui est monté autour d'une extrémité de la cartouche et qui est prévu pour coopérer avec le support de cartouche. Cet élément de couplage laisse la plus grande partie de la cartouche exposée, tant que celle-ci n'est pas montée au sein du stylo injecteur, d'où un risque non négligeable de bris de cette cartouche dont le contenu est alors perdu, ce qui empêche le traitement.

Il est d'autre part connu de WO-A-2013/124118 d'utiliser des mâchoires ménagées à l'extrémité de bras flexibles pour retenir une cartouche de principe actif à l'intérieur d'un support d'un stylo injecteur. Ce matériel est relativement complexe à utiliser, notamment par un patient qui peut être affaibli ou peu familier de ces manipulations.

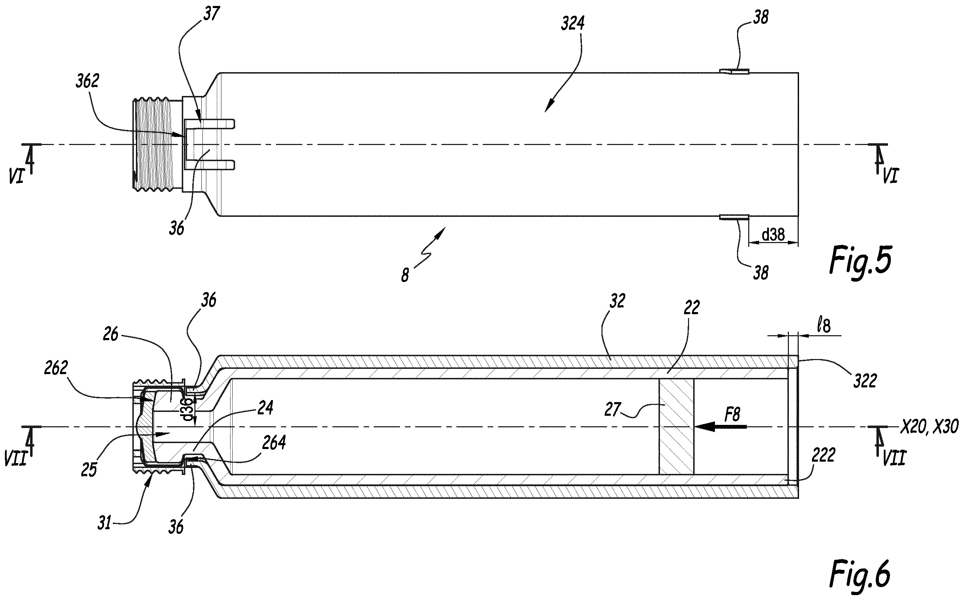

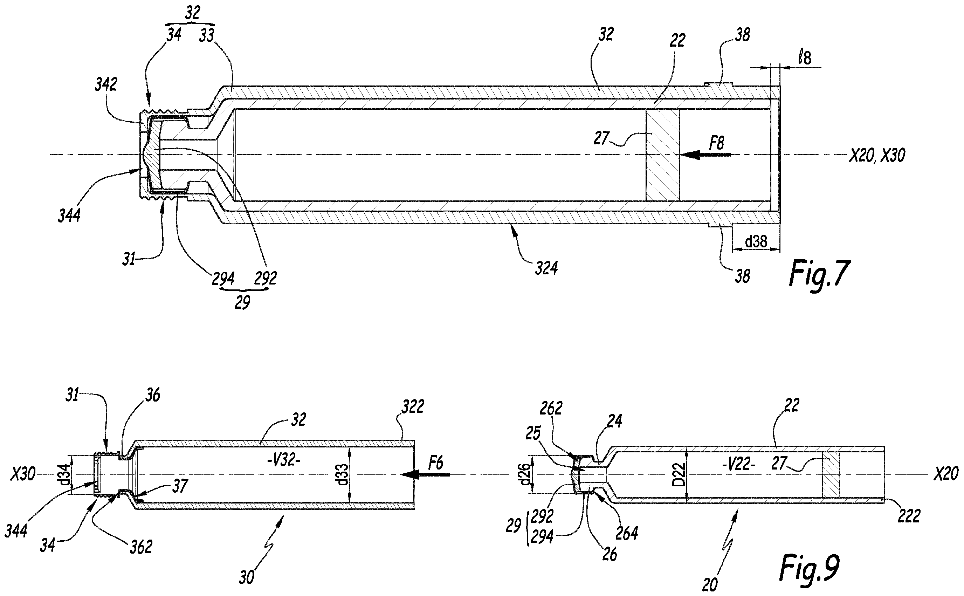

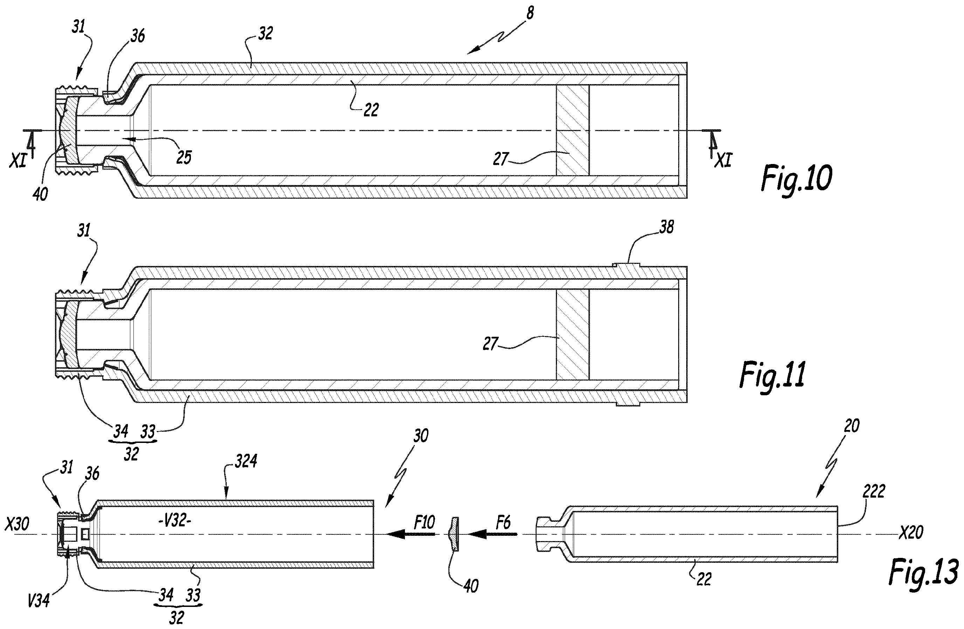

Par ailleurs, WO-A-2012/017063 enseigne, dans un mode de réalisation représenté à la figure 6, d'utiliser une bague fendue pour retenir un récipient dans un boîtier. Cette bague fendue peut être comprimée pour extraire le récipient du boîtier, de sorte que le blocage obtenu n'est pas irréversible. Dans un autre mode de réalisation représenté aux figures 9 et 10, l'immobilisation d'un récipient dans un boîtier est effectuée par l'arrière. Ceci impose que les dimensions du récipient soient parfaitement maitrisées pour qu'il puisse coopérer avec des moyens de fixation prévus à l'arrière du boîtier, ce qui n'est en pratique pas possible compte-tenu des tolérances de fabrication des récipients et des boîtiers. Enfin, dans d'autres modes de réalisation représentés aux figures 14 à 18, un cavalier est rapporté sur un boîtier pour coincer un récipient dans un boîtier. Tout comme la bague fendue du mode de réalisation de la figure 6, ce cavalier est une pièce distincte du corps du boîtier, qu'il convient de produire et approvisionner à part et dont la mise en place s'avère difficile dans le cadre d'une production industrielle.

Enfin, WO-A-2011/092326 divulgue un boîtier de réception d'un récipient de type carpule, ce boîtier étant équipé d'éléments de fixation conformés pour ne pas interférer avec le récipient tant qu'une aiguille creuse n'a pas été montée sur le boîtier pour exercer, sur une extension de chaque élément de fixation, un effort centripète pour rabattre radialement vers l'intérieur cet élément de fixation. Ainsi, aucun blocage d'un récipient dans le boîtier ne peut être obtenu tant que l'aiguille creuse n'est pas en place. En outre, le blocage obtenu n'est pas irréversible puisqu'il suffit de démonter l'aiguille creuse, ce qui peut avoir lieu en pratique après chaque injection, pour que les éléments de fixation reprennent leur géométrie d'origine et que le récipient soit libéré.

C'est à ces inconvénients qu'entend plus particulièrement remédier l'invention en proposant une nouvelle approche pour monter un récipient tel qu'une capsule ou carpule sur un stylo injecteur.

A cet effet, l'invention concerne un boîtier de montage d'un récipient de forme allongée sur un stylo injecteur dans lequel ce boîtier de montage comprend un corps de forme allongée qui s'étend selon un axe longitudinal et qui définit un volume intérieur de réception complète du récipient. Le corps est pourvu, sur l'extérieur d'une première extrémité axiale, d'au moins un premier organe mécanique de fixation d'une aiguille et au voisinage d'une deuxième extrémité axiale opposée à la première extrémité axiale, d'au moins un deuxième organe mécanique de fixation du boîtier sur un stylo injecteur. Conformément à l'invention, le boîtier comprend, au voisinage de sa première extrémité, au moins un troisième organe mécanique de blocage axial irréversible d'un récipient en position complètement contenue dans le volume intérieur du corps. En outre, le troisième organe est une languette élastiquement déformable du boîtier, qui est monobloc avec le corps, lui-même monobloc, et destinée à assurer un blocage irréversible du récipient en position dans le volume intérieur du corps par une retenue axiale d'une collerette du récipient.

Grâce à l'invention, le boîtier permet de protéger efficacement le récipient, tout particulièrement lorsqu'il s'agit d'une carpule, sur toute sa longueur, tout en évitant que le stylo injecteur avec lequel est prévu de coopérer le récipient soit équipé d'un organe support de forme relativement complexe, puisque c'est le boîtier lui-même qui assure la fixation du récipient sur une embase du stylo injecteur. En outre, le deuxième organe mécanique peut être configuré en fonction du stylo injecteur sur lequel est prévu pour être monté le récipient, ce qui évite des erreurs de manipulation puisque le boîtier sert alors de détrompeur pour le montage du récipient sur le stylo injecteur. Enfin, le troisième organe mécanique assure le caractère indémontable de l'assemblage entre le boîtier et un récipient et, par voie de conséquence, la pérennité de la protection conférée par le boîtier à un récipient. En particulier, le fait que ce troisième organe mécanique est constitué par une languette élastiquement déformable du boîtier évite d'avoir recours à un organe annexe tel qu'une bague fendue ou un cavalier.

Selon des aspects avantageux mais non obligatoires de l'invention, un tel boîtier peut incorporer une ou plusieurs des caractéristiques suivantes, prises dans toute combinaison techniquement admissible :
- En configuration non contrainte, la languette s'étend à une distance radiale de l'axe longitudinal inférieure à la moitié du diamètre intérieur de la première extrémité axiale.
- La languette comprend un bord opposé à la deuxième extrémité axiale du boîtier et qui est destiné à bloquer axialement, de façon irréversible, la collerette du récipient.
- La languette est monobloc avec le corps.
- La languette est entourée par une découpe en forme de U à fond plat dont le fond délimite le bord de la languette opposé à la deuxième extrémité axiale.
- Le boîtier comprend deux languettes diamétralement opposées par rapport à l'axe longitudinal.
- La ou chaque languette est configurée pour être repoussée radialement vers l'extérieur de la première extrémité axiale par une partie d'un récipient en cours de montage irréversible dans le boîtier.
- Le corps est au moins en partie réalisé dans un matériau transparent.
- Le premier organe mécanique de fixation comprend un filetage et/ou un relief de verrouillage à baïonnette
- Le deuxième organe mécanique de fixation comprend un filetage, un taraudage et/ou un relief de verrouillage à baïonnette.

Par ailleurs, l'invention concerne un ensemble formant réservoir de produit injectable pour un stylo injecteur, cet ensemble comprenant un récipient allongé contenant un produit injectable, ainsi qu'un boîtier tel que mentionné ci-dessus, alors que le récipient est logé complètement dans le volume intérieur du corps de ce boîtier.

Un tel ensemble peut être manipulé de façon unitaire, notamment pour son transport et son montage sur un stylo injecteur, alors que le récipient est efficacement protégé contre les chocs et les risques de pollution du fait qu'il est complètement logé dans le volume intérieur du corps du boîtier.

Selon des aspects avantageux mais non obligatoires de l'invention, un tel ensemble peut incorporer une ou plusieurs des caractéristiques suivantes prises selon toute combinaison techniquement admissible :
- La deuxième extrémité s'étend, le long de l'axe longitudinal du corps et sur une distance non nulle, au-delà du récipient.
- Le récipient est équipé d'une collerette, qui entoure une embouchure du récipient, alors que la languette élastiquement déformable est disposée en regard de, ou en appui contre, une surface axiale de la collerette, orientée à l'opposé de l'embouchure du récipient.
- Le récipient est équipé d'un bouchon et d'une coiffe entourant le bouchon, qui sont insérés dans la première extrémité axiale du boîtier.
- En variante, le boîtier est pourvu d'un renfoncement de réception d'un bouchon indépendamment du récipient, alors que le récipient est dépourvu de coiffe entourant le bouchon.

Enfin, l'invention concerne un stylo injecteur, destiné à être utilisé pour injecter un principe actif dans le corps d'un patient et qui comprend une embase de prise en main par un utilisateur, ainsi qu'un réservoir amovible de produit injectable. Selon l'invention, ce réservoir de produit injectable est formé par un ensemble tel que mentionné ci-dessus, alors que le deuxième organe mécanique du corps du boîtier est en prise avec un organe mécanique complémentaire ménagé sur l'embase.

L'invention sera mieux comprise et d'autres avantages de celle-ci apparaîtront plus clairement à la lumière de la description qui va suivre, de deux modes de réalisation d'un boîtier, d'un ensemble formant réservoir et d'un stylo injecteur conformes à son principe, donnée uniquement à titre d'exemple et faite en référence aux dessins annexés, dans lesquels :
- la figure 1 est une vue en perspective d'un stylo injecteur conforme à l'invention, dans une configuration de stockage ou de transport ;
- la figure 2 est une vue en perspective du stylo de la figure 1 dans une configuration de pré-utilisation ;
- la figure 3 est une vue en perspective analogue à la figure 1 dans une configuration de changement d'un ensemble formant réservoir du produit injectable pour le stylo injecteur des figures 1 et 2 ;
- la figure 4 est une vue en perspective, selon un autre angle, de l'ensemble formant réservoir visible aux figures 2 et 3 ;
- la figure 5 est une vue de côté de l'ensemble formant réservoir de la figure 4 ;
- la figure 6 est une coupe selon la ligne VI-VI à la figure 5 ;
- la figure 7 est une coupe selon la ligne VII-VII à la figure 6 ;
- la figure 8 est une vue en perspective éclatée de l'ensemble formant réservoir en cours de montage ;
- la figure 9 est une coupe selon le plan IX à la figure 8, à plus petite échelle que les figures 6 et 7 ;
- la figure 10 est une coupe analogue à la figure 6 pour un ensemble formant réservoir conforme à un deuxième mode de réalisation de l'invention ;
- la figure 11 est une coupe selon la ligne XI-XI à la figure 10 ;
- la figure 12 est une vue en perspective éclatée de l'ensemble formant réservoir des figures 10 et 11 en cours de montage, et
- la figure 13 est une coupe selon le plan XIII à la figure 12, à plus petite échelle que les figures 10 et 11.

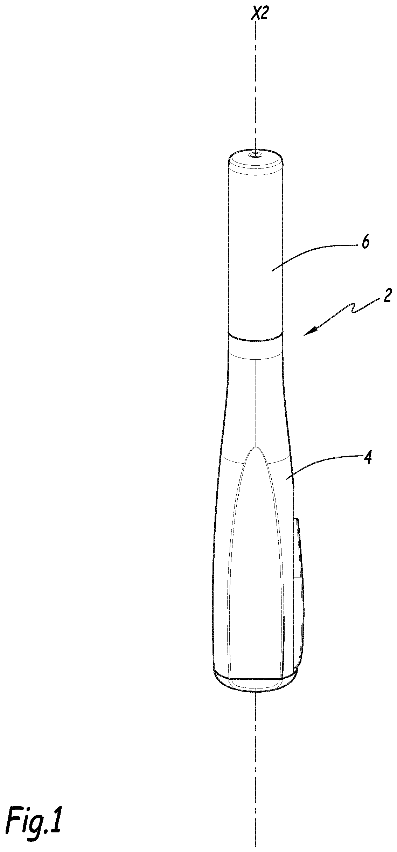

Le stylo injecteur 2 visible aux figures 1 à 3 s'étend selon un axe longitudinal X2 et comprend une embase 4, destinée à être prise en main par un utilisateur lors d'une injection, ainsi qu'un capuchon 6 rapporté sur l'embase 4 et qui, lorsqu'il est en place sur cette embase 4, isole de l'extérieur un ensemble 8 formant réservoir, lui-même monté sur l'embase 4, ainsi qu'il ressort des explications qui suivent.

L'ensemble 8 contient un produit injectable, notamment un médicament contenant un produit actif destiné à traiter une maladie nécessitant un traitement à prise régulière, telle que le diabète ou le nanisme.

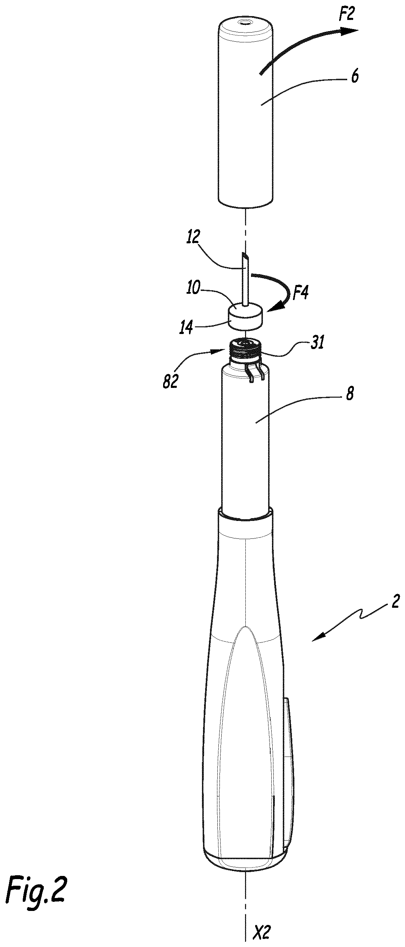

Comme représenté à la figure 2, lorsqu'il convient d'utiliser le stylo injecteur 2, le capuchon 8 est retiré, ce que représente la flèche F2, et un élément jetable 10, comprenant une aiguille 12, est monté à l'extrémité distale 82 de l'ensemble 8, ce que représente la flèche F4. Pour ce faire, l'extrémité distale 82 est pourvue d'un filetage extérieur 31, alors qu'une partie de base 14 de l'ensemble 10 est équipée d'un taraudage interne de géométrie correspondante, non visible à la figure 2. Ainsi, un élément jetable 10 peut être vissé sur l'extrémité distale 82 avant chaque utilisation du stylo injecteur 2, son aiguille 12 étant alors alignée sur l'axe X2.

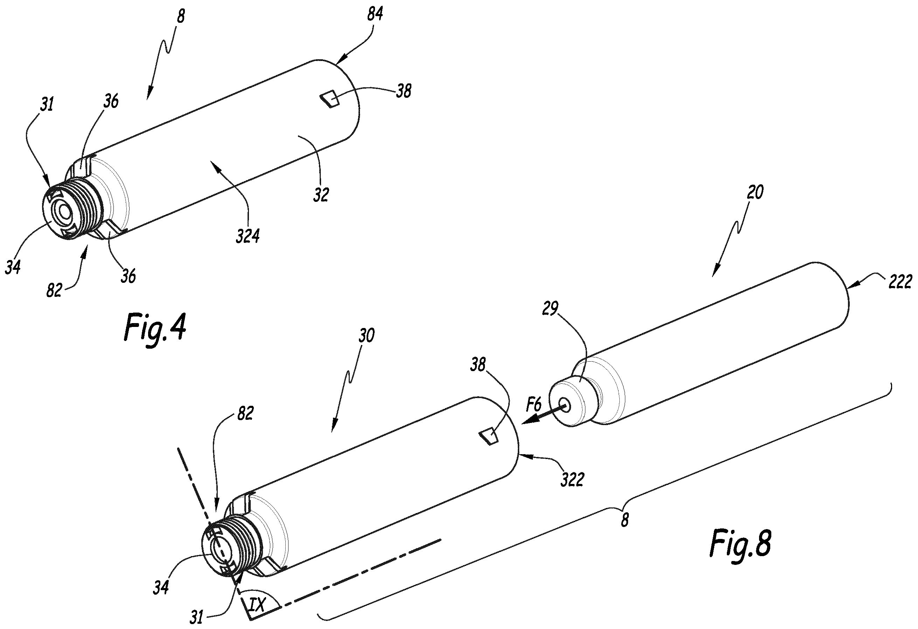

L'ensemble 8 formant réservoir est représenté seul aux figures 4 à 9. Il est formé de deux éléments, à savoir une carpule ou cartouche 20 et un boîtier ou enveloppe 30 qui est prévu(e) pour, d'une part, contenir la carpule 20 et, d'autre part, permettre, au niveau de l'extrémité distale 82, sa fixation avec l'élément jetable 10 et, au niveau d'une extrémité proximale 84 de l'ensemble 8, sa fixation sur l'embase 4.

La carpule 20 est en verre et forme un récipient de forme allongée centré sur un axe longitudinal X20 et présentant un corps cylindrique 22 à section circulaire dont le diamètre extérieur D22 est compris entre 8,55 et 12 mm. En variante, la carpule 20 peut être en matière plastique. Cette carpule est pourvue d'un col 24 définissant un goulot 25 par lequel il est possible de remplir la carpule ou d'y prélever un produit injectable et qui est entouré par une collerette 26 dont on note 262 une surface annulaire entourant le débouché du goulot 25 et 264 une surface annulaire orientée à l'opposé de la surface 262 et tournée vers une extrémité 222 du corps 22 opposée au goulot 25.

Dans ce qui suit, une direction est dite axiale lorsqu'elle est parallèle à un axe et radiale lorsqu'elle est perpendiculaire et sécante à cet axe. Une surface est dite axiale lorsqu'elle est perpendiculaire à une direction axiale et radiale lorsqu'elle est perpendiculaire à une direction radiale.

Les surfaces 262 et 264 sont des surfaces axiales par rapport à l'axe X20.

L'extrémité 222 du corps 22 est ouverte et un piston 27 est logé dans le corps 22, à travers cette extrémité 222, avec une possibilité de coulissement axial le long de l'axe X20. Le coulissement axial du piston 27 dans le corps 22 est commandé par des moyens intégrés à l'embase 4, qui sont connus en soi et ne sont pas décrits en détail ici.

Un dispositif de bouchage 29 est monté sur le corps 22, du côté du col 24 et comprend un bouchon 292 formé par une rondelle élastomère qui est circulaire et plaquée sur la surface 262. Le dispositif de bouchage 29 comprend également une coiffe 294, qui peut être en aluminium ou réalisée par moulage de matière plastique et qui immobilise le bouchon 292 sur la collerette 26.

Le boîtier 30 est centré sur un axe longitudinal X30 et comprend un corps 32 réalisé par moulage de matière plastique, par exemple du polycarbonate.

Le corps 32 définit un volume intérieur V32 dans lequel peut être reçue complètement la carpule 20. En d'autres termes, la carpule 20 peut être logée dans le volume V32 sans dépasser à l'extérieur du corps 32 du boîtier 30.

Le corps 32 comprend une partie principale 33 dont le diamètre intérieur d33 est légèrement supérieur au diamètre D22. Par légèrement supérieur on entend que le diamètre d33 est inférieur à 110% du diamètre D22. Le corps 32 comprend également une tête 34, monobloc avec la partie principale 33 et destinée à recevoir la collerette 26 équipée du dispositif de bouchage 29. La tête 34 forme l'extrémité distale 82 de l'ensemble 8.

A la jonction entre la partie principale 33 du corps 32 et la tête 34, le boîtier 30 est pourvu de deux languettes 36 élastiquement déformables et qui sont également monobloc avec le reste du corps 32. Les languettes 36 sont chacune entourées par une découpe 37 en forme de U à fond plat. Comme visible à la figure 6, les languettes 36 sont diamétralement opposées par rapport à l'axe X30.

On note 322 l'extrémité ouverte du corps 32 opposée à la tête 84 et 362 le bord de chaque languette 36 orientée à l'opposé de l'extrémité 322. L'extrémité 322 forme l'extrémité proximale 84 de l'ensemble 8.

Le corps 32 est dépourvu de fenêtre ou d'ouverture. En d'autres termes, le corps 32 est continu autour de l'axe X30, de sorte qu'il isole complètement le volume V32 de l'extérieur, sauf au niveau de l'extrémité ouverte 322.

La tête 34 est pourvue d'un fond 342 qui définit une ouverture centrale 344 centrée sur l'axe X30 et qui donne accès au bouchon 292 en configuration montée de la carpule 20 dans le boîtier 30. Le diamètre intérieur d34 de la tête 34, défini dans une partie de cette tête 34 comprise entre le fond 342 et les bords 362 des languettes 36 est légèrement supérieur au diamètre extérieur D26 de la collerette 26 entourée de la coiffe 294, avec un rapport d34/D26 inférieur à 1,05.

Comme visible notamment aux figures 5 et 6, chaque languette 36 s'étend, parallèlement à l'axe X30, de l'extrémité 322 vers le fond 342, avec son bord 362 tourné vers ce fond.

En configuration non contrainte, c'est-à-dire en l'absence d'effort extérieur appliqué sur celles-ci, les languettes 36 s'étendent à une distance radiale d36 de l'axe X30 inférieure à la moitié du diamètre d34. En d'autres termes, les languettes 36 font saillie radialement vers l'intérieur du volume V32 par rapport au reste de la tête 34.

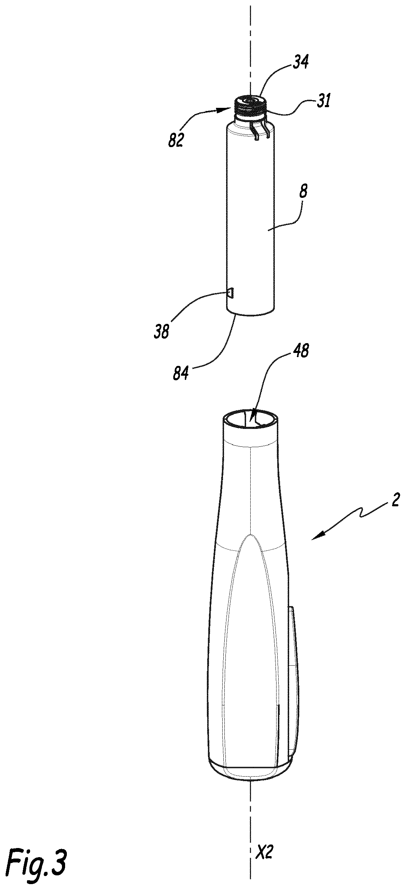

Par ailleurs, et comme il ressort plus particulièrement des figures 4, 5, 7 et 8, le corps 32 porte, sur sa surface radiale externe 324 et au voisinage de l'extrémité 322, deux pions 38 en saillie radialement par rapport à la surface 324 et qui sont diamétralement opposés. La distance axiale d38 entre les pions 38 et l'extrémité 322 est inférieure à 10 mm. Chaque pion 38 est destiné à coopérer avec une gorge de l'embase 4, dont une seule est visible à la figure 3 avec la référence 48. Chaque gorge 48 comprend une branche longitudinale, parallèle à l'axe X2 et une branche transversale ou inclinée par rapport à cet axe. Ainsi, les éléments 38 et 48 constituent ensemble un système de verrouillage à baïonnette du boîtier 30, et par là même de l'ensemble 8, sur l'embase 4.

Lorsqu'il convient de monter la carpule 20 dans le boîtier 30, les axes X20 et X30 sont alignés et la carpule 20 est poussée dans le volume V32, dans le sens de la flèche F6 aux figures 8 et 9 jusqu'à ce que le dispositif de bouchage 29 vienne en butée contre le fond 342 de la tête 34. Au passage, la coiffe 294 repousse radialement les languettes 36 vers l'extérieur de la tête 34, ces languettes pouvant s'écarter radialement de l'axe X30 du fait de leur élasticité. Lorsque la coiffe 294 parvient au contact du fond 342, la collerette 26 a dépassé axialement, le long de l'axe X30, les languettes 36 qui sont alors disposées, le long de cet axe, au niveau du col 24, avec leurs bords respectifs 362 en regard de ou en appui contre la surface 264 de la collerette 26 et de la partie de la coiffe 294 qui recouvre cette surface. Ainsi, la carpule 20 est verrouillée axialement dans le corps 32 du boîtier 30, de façon irréversible.

L'ensemble 8 est ainsi constitué et peut être manipulé par un utilisateur de façon unitaire.

Comme cela ressort des figures 6 et 7, l'extrémité 222 du corps 22 est en retrait, à l'intérieur du corps 32, En pratique, une distance ℓ8 sépare les extrémités 222 et 322 le long des axes X20 et X30. Ainsi, l'extrémité 322 s'étend, le long de ces axes, au-delà de la carpule 20, laquelle est efficacement protégée contre les chocs, notamment lorsque l'ensemble 8 formant réservoir est posé sur une surface verticale avec son extrémité distale 82 orientée vers le haut.

En pratique, lorsqu'un ensemble 8 a été utilisé sur le stylo injecteur 2, au point que sa carpule 20 est vide ou quasi vide, cet ensemble 8 peut être retiré de l'embase 4 grâce à un mouvement de rotation autour des axes X2, X20 et X30 alors confondus et de translation parallèle à cet axe permettant de déverrouiller le système à baïonnette formé des éléments 38 et 48. Ensuite, un nouvel ensemble formant réservoir 8, dont la carpule 20 est pleine, peut être monté sur l'embase 4, par un nouveau mouvement de translation/rotation activant le système à baïonnette.

Dans ce cas, l'ensemble 8 préalablement démonté du stylo injecteur 2 est jeté. En d'autres termes, les éléments 20 et 30 sont jetables après utilisation, ce qui est cohérent avec le fait que le blocage de la carpule 20 dans le boîtier 30 est irréversible.

Le filetage extérieur 31 est formé sur la périphérie de la tête 34 et permet de monter successivement, sur l'ensemble 8 en place sur l'embase 4, des éléments jetables 10 à chaque fois qu'une dose du produit contenu dans la carpule 20 doit être injectée à un patient. Le montage de l'élément jetable 10 sur l'extrémité distale 82 de l'ensemble 8 formé par la tête 34 a pour effet de perforer le bouchon 292 à travers l'ouverture 344. Lorsque l'élément jetable 10 est en place sur l'ensemble 8, un effort de poussée, dirigé vers la tête 34, est exercé sur le piston 27 comme représenté par les flèches F8 aux figures 6 et 7, afin de chasser une partie du produit présent dans le volume intérieur V22 du corps 22 en direction de l'aiguille 12 de l'élément jetable 10.

Le corps 32 est transparent, au moins au niveau de sa partie principale qui entoure le corps 22. Ceci permet de visualiser à travers le corps 32 une étiquette apposée sur le corps 22, afin de vérifier l'adéquation du produit contenu dans le volume V22 par rapport au traitement prescrit au patient et au type de stylo injecteur 2 utilisé.

Le corps 32 n'est pas forcément transparent en totalité. Seule une partie de celui-ci peut être transparente, dans une zone permettant de visualiser une étiquette apposée sur la carpule 20, ainsi que le niveau de produit restant dans cette carpule.

Par ailleurs, la géométrie et le positionnement des pions 38 peut être configuré(e) en fonction de la géométrie et du positionnement des gorges 48. Ceci permet d'appairer un type d'élément 8 formant réservoir avec un type de stylo injecteur 2. On évite ainsi les risques de fausse manipulation qui consisteraient à monter sur un stylo injecteur 2 un ensemble 8 qui n'est pas adapté à ce stylo injecteur, par exemple car le volume V22 a un diamètre différent de celui pour lequel le déplacement linéaire des moyens de poussée sur le piston 27 est calculé. En particulier, les pions 38 et gorges 48 ne sont pas forcement diamétralement opposés.

Dans le deuxième mode de réalisation de l'invention représenté aux figures 10 à 12, les éléments analogues à ceux du premier mode de réalisation portent les mêmes références. Dans ce qui suit, on décrit principalement ce qui distingue ce mode de réalisation du précédent.

Dans ce mode de réalisation, la carpule 20 est dépourvue de dispositif de bouchage avant son montage dans le boîtier 30. En particulier, aucune coiffe comparable à la coiffe 294 n'est utilisée dans ce deuxième mode de réalisation.

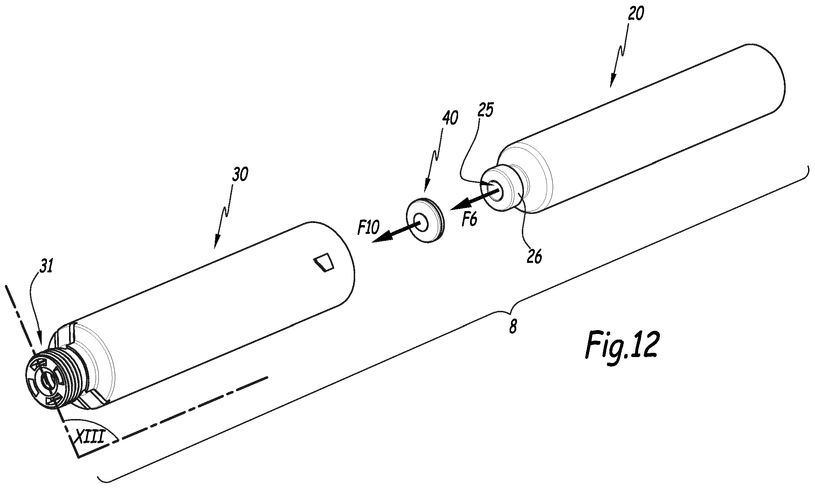

Dans ce mode de réalisation, un bouchon 40 est mis en place dans la tête 34 du boîtier 30 avant introduction de la carpule 20 dans le volume V32. Plus précisément, la tête 34 forme un volume V34 de réception et de coincement du bouchon 40, comme visible à la figure 13. Le volume V34 est une partie du volume V32. L'assemblage de l'ensemble 8 formant réservoir selon ce deuxième mode de réalisation est effectué en insérant tout d'abord le bouchon 40 dans la tête 34, dans le sens des flèches F10 aux figures 12 et 13, puis en insérant la carpule 20 dépourvue de dispositif de bouchage et de piston dans le volume V32, comme représenté par les flèches F6 aux figures 12 et 13.

En variante, le bouchon 40 peut être surmoulé dans le boîtier 30 lors de sa fabrication. Il n'est alors pas nécessaire de procéder à l'insertion selon les flèches F10.

On constitue alors un ensemble 8 formant réservoir qui est vide et qui peut être rempli à travers l'extrémité 222 du corps 22 de la carpule 20 avant mise en place du piston 27.

Une fois rempli et équipé de son piston, l'ensemble 8 formant réservoir de ce deuxième mode de réalisation est utilisable comme celui du premier mode de réalisation.

En particulier, il comprend un filetage 31 permettant le montage d'un élément jetable 10 et des pions 38 permettant de le fixer sur l'embase 4 d'un stylo injecteur 2.

Dans ce cas, comme la carpule 20 est remplie en étant déjà en place dans le corps 30, l'étiquetage du produit peut être effectué sur la surface radiale externe 324 du corps 32, lequel n'est pas nécessairement transparent, même si cela est avantageux pour permettre de visualiser le niveau de produit restant dans la carpule 20, comme pour le premier mode de réalisation.

Quel que soit le mode de réalisation, le filetage 31 constitue un organe mécanique de fixation de l'aiguille 12 sur le boîtier 30. En variante, d'autres types d'organes mécaniques peuvent être envisagés pour remplir cette fonction, notamment des ergots permettant un montage de l'élément jetable 20 par un système à baïonnette.

De même, quel que soit le mode de réalisation, les pions 38 constituent des organes mécaniques de fixation du boîtier 30 sur l'embase 4. En variante, ils peuvent être remplacés par d'autres organes mécaniques assurant la même fonction, par exemple un filetage ménagé sur la surface 324, pour autant que l'embase 4 est équipée d'un taraudage correspondant. Par ailleurs, si le corps 32 dépasse suffisamment du corps 22 du côté de l'extrémité proximale 84 de l'élément 8, un taraudage peut être prévu sur la surface intérieure du corps 32 alors qu'un filetage correspondant est prévu sur l'embase 4.

Dans le ou les système(s) à baïonnette utilisé(s), les reliefs mâles et femelles, c'est-à-dire les pions 38 et les gorges 48, sont disposés, au choix sur le boîtier 30 ou sur l'embase 40.

## Revendications

1. Boîtier (30) de montage d'un récipient (20) de forme allongée sur un stylo injecteur (2), ce boîtier de montage comprenant un corps (32) de forme allongée qui s'étend selon un axe longitudinal (X30) et qui définit un volume intérieur (V32) de réception complète du récipient, alors que le corps est pourvu, sur l'extérieur d'une première extrémité axiale (34), d'au moins un premier organe mécanique (31) de fixation d'une aiguille (12) et, au voisinage d'une deuxième extrémité axiale (322) opposée à la première extrémité axiale, d'au moins un deuxième organe mécanique (38) de fixation du boîtier sur un stylo injecteur (2) ; **caractérisé en ce que**
- le boîtier comprend, au voisinage de la première extrémité axiale (34), au moins un troisième organe mécanique (36) de blocage axial irréversible d'un récipient (20) en position complètement contenue dans le volume intérieur (V32) du corps (32) et
- le troisième organe est une languette (36) élastiquement déformable du boîtier (30), qui est monobloc avec le corps (32), lui-même monobloc, et destinée à assurer un blocage du récipient (20) en position dans le volume intérieur (V32) du corps (32) par une retenue axiale d'une collerette (26) du récipient.

2. Boîtier selon la revendication 1, **caractérisé en ce que**, en configuration non contrainte, la languette (36) s'étend à une distance radiale (d36) de l'axe longitudinal (X30) inférieure à la moitié du diamètre intérieur (d34) de la première extrémité axiale (34).

3. Boîtier selon l'une des revendications précédentes, **caractérisé en ce que** la languette comprend un bord (362) opposé à la deuxième extrémité axiale (322) du boîtier et qui est destiné à bloquer axialement, de façon irréversible, la collerette (26) du récipient (20).

4. Boîtier selon la revendication 3, **caractérisé en ce que** la languette est entourée par une découpe (37) en forme de U à fond plat dont le fond délimite le bord (362) de la languette opposé à la deuxième extrémité axiale (322).

5. Boîtier selon l'une des revendications précédentes, **caractérisé en ce qu'**il comprend deux languettes (36) diamétralement opposées par rapport à l'axe longitudinal (X30).

6. Boîtier selon l'une des revendications précédentes, **caractérisé en ce que** la ou chaque languette (36) est configurée pour être repoussée radialement vers l'extérieur de la première extrémité axiale (34) par une partie (294 ; 26) d'un récipient en cours de montage irréversible dans le boîtier (30).

7. Boîtier selon l'une des revendications précédentes, **caractérisé en ce que** le corps (32) est au moins en partie réalisé dans un matériau transparent.

8. Boîtier selon l'une des revendications précédentes, **caractérisé en ce que** :
- le premier organe mécanique de fixation comprend un filetage (31) et/ou un relief de verrouillage à baïonnette et/ou
- le deuxième organe mécanique de fixation comprend un filetage et/ou un taraudage et/ou un relief (38) de verrouillage à baïonnette.

9. Ensemble (8) formant réservoir de produit injectable pour stylo injecteur et comprenant un récipient allongé (20) contenant un produit injectable, **caractérisé en ce qu'**il comprend en outre un boîtier (30) selon l'une des revendications précédentes et **en ce que** le récipient est logé complètement dans le volume intérieur (V32) du corps (32) du boîtier.

10. Ensemble selon la revendication 9, **caractérisé en ce que** la deuxième extrémité (322) s'étend, le long de l'axe longitudinal (X30) du corps et sur une distance (f8) non nulle, au-delà du récipient (20).

11. Ensemble selon l'une des revendications 9 ou 10, **caractérisé en ce que** le récipient (20) est équipé d'une collerette (26), qui entoure une embouchure (25) du récipient et **en ce que** la languette (36) élastiquement déformable est en regard de, ou en appui contre, une surface axiale (264) de la collerette, orientée à l'opposé de l'embouchure du récipient.

12. Ensemble selon l'une des revendications 9 à 11, **caractérisé en ce que** le récipient (20) est équipé d'un bouchon (292) et d'une coiffe (294) entourant le bouchon, qui sont insérés dans la première extrémité axiale (34) du boîtier (32).

13. Ensemble selon l'une des revendications 9 à 11, **caractérisé en ce que** le boîtier (30) est pourvu d'un renfoncement (34) de réception d'un bouchon (40) indépendamment du récipient (20) et **en ce que** le récipient est dépourvu de coiffe entourant le bouchon.

14. Ensemble selon l'une des revendications 9 à 13, **caractérisé en ce que** le récipient allongé (20) et le boîtier (30) sont jetables après utilisation.

15. Stylo injecteur (2), destiné à être utilisé pour injecter un principe actif dans le corps d'un patient et comprenant :
- une embase (4) de prise en main par un utilisateur,
- un réservoir amovible de produit injectable,
**caractérisé en ce que** le réservoir amovible de produit injectable est formé par un ensemble (8) selon l'une des revendications 9 à 13 et **en ce que** le deuxième organe mécanique (38) du corps (32) du boîtier (30) est en prise avec un organe mécanique complémentaire (48) ménagé sur l'embase (4).

## Patentansprüche

1. Gehäuse (30) zum Montieren eines Behälters (20) langgestreckter Form an einen Injektionsstift (2), wobei dieses Montagegehäuse einen langgestreckten Körper (32) umfasst, der sich gemäß einer Längsachse (X30) erstreckt und der ein Innenvolumen (V32) zur vollständigen Aufnahme des Behälters definiert, wobei der Körper außerhalb eines ersten axialen Endes (34) mit mindestens einem ersten mechanischen Element (31) zum Befestigen einer Nadel (12) und in der Nähe eines zweiten axialen Endes (322), entgegensetzt zum ersten axialen Ende, mit mindestens einem zweiten Element (38) zum Befestigen des Gehäuses auf einem Injektionsstift (2) versehen ist; **dadurch gekennzeichnet, dass**
- das Gehäuse in der Nähe des ersten axialen Endes (34) mindestens ein drittes mechanisches Element (36) zum irreversiblen axialen Festlegen eines Behälters (20) in einer vollständig in dem Innenvolumen (V32) des Körpers (32) enthaltenen Position umfasst und
- das dritte Element eine elastisch verformbare Zunge (36) des Gehäuses (30) ist, die einstückig mit dem Körper (32), der selbst in einem Stück ist, ausgebildet ist und vorgesehen ist, ein Festlegen des Behälters (20) in Position in dem Innenvolumen (V32) des Körpers (32) durch ein axiales Halten eines Bunds (26) des Behälters sicherzustellen.

2. Gehäuse nach Anspruch 1, **dadurch gekennzeichnet, dass** in einer nicht gespannten Konfiguration sich die Zunge (36) in einer radialen Entfernung (d36) von der Längsachse (X30) kleiner als die Hälfte des Innendurchmessers (d34) des ersten axialen Endes (34) erstreckt.

3. Gehäuse nach einem der vorhergehenden Ansprüche, **dadurch gekennzeichnet, dass** die Zunge einen Rand (362) entgegengesetzt zu dem zweiten axialen Ende (322) des Gehäuses umfasst und der vorgesehen ist, axial in irreversibler Weise den Bund (26) des Behälters (20) zu blockieren.

4. Gehäuse nach Anspruch 3, **dadurch gekennzeichnet, dass** die Zunge von einem Ausschnitt (37) in U Form mit flachem Grund umgeben ist, dessen Grund den Rand (362) der Zunge entgegengesetzt zu dem zweiten axialen Ende (322) begrenzt.

5. Gehäuse nach einem der vorhergehenden Ansprüche, **dadurch gekennzeichnet, dass** es zwei Zungen (36) umfasst, die diametral in Bezug auf die Längsachse (X30) gegenüberliegen.

6. Gehäuse nach einem der vorhergehenden Ansprüche, **dadurch gekennzeichnet, dass** die oder jede Zunge (36) ausgebildet ist, radial zum ersten axialen Ende (34) durch einen Teil (294; 26) eines Behälters während der irreversiblen Montage des Behälters (30) nach außen gedrückt zu werden.

7. Gehäuse nach einem der vorhergehenden Ansprüche, **dadurch gekennzeichnet, dass** der Körper (32) mindestens teilweise aus einem transparenten Material hergestellt ist.

8. Gehäuse nach einem der vorhergehenden Ansprüche, **dadurch gekennzeichnet, dass**:
- das erste mechanische Element zum Befestigen ein Gewinde (31) oder ein Bajonett-Verriegelungsverschluss umfasst und/oder
- das zweite mechanische Element zum Befestigen ein Außengewinde und/oder ein Innengewinde und/oder ein Bajonett-Verriegelungsverschluss (38) umfasst.

9. Anordnung (8), die ein Reservoir an injizierbarem Stoff für einen Injektionsstift bildet und einen einen injizierbaren Stoff enthaltenden langgestreckten Behälter (20) umfasst, **dadurch gekennzeichnet, dass** sie außerdem ein Gehäuse (30) nach einem der vorhergehenden Ansprüche umfasst und dass der Behälter vollständig in dem Innenvolumen (V32) des Körpers (32) des Gehäuses aufgenommen ist.

10. Anordnung nach Anspruch 9, **dadurch gekennzeichnet, dass** das zweite Ende (322) sich entlang der Längsachse (X30) des Körpers und über eine Entfernung (l8), die nicht null ist, über den Behälter (20) hinaus erstreckt.

11. Anordnung nach einem der Ansprüche 910, **dadurch gekennzeichnet, dass** der Behälter (20) mit einem Bund (26) ausgerüstet ist, der einen Einlass (25) des Behälters umgibt, und dass die elastisch verformbare Zunge (36) gegenüber einer oder in Anlage gegen eine axiale Fläche (264) des Bunds liegt, die entgegengesetzt zu dem Einlass des Behälters gerichtet ist.

12. Anordnung nach einem der Ansprüche 9 bis 11, **dadurch gekennzeichnet, dass** der Behälter (20) mit einem Stopfen (292) und einem Klemmelement (294), das den Stopfen umgibt, ausgerüstet ist, die in das erste axiale Ende (34) des Gehäuses (32) eingesetzt sind.

13. Anordnung nach einem der Ansprüche 9 bis 11, **dadurch gekennzeichnet, dass** das Gehäuse (30) mit einer Vertiefung (34) zur Aufnahme eines Stopfens (40) unabhängig vom Behälter (20) versehen ist und dass der Behälter kein den Stopfen umgebenden Klemmelement aufweist.

14. Anordnung nach einem der Ansprüche 9 bis 11, **dadurch gekennzeichnet, dass** der langgestreckt Behälter (20) und das Gehäuse (30) nach der Benutzung wegwerfbar sind.

15. Injektionsstift (2), der vorgesehen ist, für eine Injektion eines aktiven Stoffs in den Körper eines Patienten verwendet zu werden, umfassend:
- einen von einem Benutzer in die Hand zu nehmenden Griffsockel (4),
- ein lösbares Reservoir für einen Injektionsstoff,
**dadurch gekennzeichnet, dass** das lösbare Reservoir für Injektionsstoff durch eine Anordnung (8) nach einem der Ansprüche 9 bis 13 gebildet wird und dass das zweite mechanische Element (38) des Körpers (32) des Gehäuses (30) im Eingriff mit einem komplementären mechanischen Element (48) ist, das an dem Sockel (4) angesetzt ist.

## Claims

1. A housing (30) for mounting an elongated container (20) on an injection pen (2), this mounting housing comprising an elongated body (32) that extends along a longitudinal axis (X30) and that defines an inner volume (V32) for receiving the container fully, while the body is provided, on the outside of a first axial end (34), with at least one first mechanical member (31) for attaching a needle (12) and, in the vicinity of a second axial end (322) opposite the first axial end, with at least one second mechanical member (38) for attaching the housing onto an injection pen (2); **characterized in that**
- the housing comprises, in the vicinity of its first axial end (34), at least one third mechanical member (36) for irreversibly locking a container (20) in position, fully contained in the inner volume (V32) of the body (32), and
- the third member is an elastically deformable tab (36) of the housing (30), which forms a single piece with the body (32), itself made in one piece, and is intended to ensure irreversible locking of the container (20) in position in the inner volume (V32) of the body (32) of the housing, by axially retaining a flange (26) of the container.

2. The housing according to claim 1, **characterized in that**, in the unstressed configuration, the tab (36) extends at a radial distance (d36) from the longitudinal axis (X30) smaller than half the inner diameter (d34) of the first axial end (34).

3. The housing according to one of the preceding claims, **characterized in that** the tab comprises an edge (362) opposite the second axial end (322) of the housing and that is intended to axially and irreversibly lock the flange (26) of the container (20).

4. The housing according to claim 3, **characterized in that** the tab is surrounded by a U-shaped cutout (37) with a flat bottom, the bottom of which delimits the edge (322) of the tab opposite the second axial end (322).

5. The housing according to one of the preceding claims, **characterized in that** it comprises two diametrically opposite tabs (36) relative to the longitudinal axis (X30).

6. The housing according to one of the preceding claims, **characterized in that** the or each tab (36) is configured to be pushed radially back toward the outside of the first axial end (34) by part (294; 26) of a container during irreversible mounting in the housing (30).

7. The housing according to one of the preceding claims, **characterized in that** the body (32) is at least partially made from a transparent material.

8. The housing according to one of the preceding claims, **characterized in that**:
- the first mechanical fastening member comprises a thread (31) and/or a bayonet locking relief, and/or
- the second mechanical fastening member comprises a thread, a tapping and/or a bayonet locking relief (38).

9. An assembly (8) forming an injectable product reservoir (20) for an injection pen, **characterized in that** it further comprises a housing (30) according to one of the preceding claims and **in that** the container is fully housed in the inner volume (V32) of the body (32) of the housing.

10. The assembly according to claim 10, **characterized in that** the second end (322) extends, along the longitudinal axis (X30) of the body and over a nonzero distance (ℓ8), past the container (20).

11. The assembly according to one of claims 9 to 10, **characterized in that** the container (20) is equipped with a flange (26), which surrounds a mouth (25) of the container, and **in that** the elastically deformable tab (36) is positioned across from, or bearing against, an axial surface (264) of the flange, oriented opposite the mouth of the container.

12. The assembly according to one of claims 9 to 11, **characterized in that** the container (20) is equipped with a stopper (292) and a cap (294) surrounding the stopper, which are inserted into the first axial end (34) of the housing (32).

13. The assembly according to one of claims 9 to 11, **characterized in that** the housing (30) is provided with a recess (34) for receiving a stopper (40) independently of the container (20), while the container has no cap surrounding the stopper.

14. The assembly according to one of claims 9 to 13, **characterized in that** the elongated container (20) and the housing (30) are disposable after use

15. An injection pen (2), intended to be used to inject an active ingredient into the body of a patient and comprising:
- a base (4) intended to be held by a user,
- a removable injectable product reservoir,
**characterized in that** the removable injectable product reservoir is formed by an assembly (8) according to one of claims 9 to 13, and **in that** the second mechanical member (38) of the body (32) of the housing (30) is engaged with a complementary mechanical member (48) arranged on the base (4).
